# EUROPEAN PATENT SPECIFICATION

(11) **EP 1 311 700 B1**
(45) Date of publication and mention of the grant of the patent: **14.10.2009**
(21) Application number: 01957004.3
(22) Date of filing: 21.08.2001
(51) Int. Cl.: C12P 13/22, C07C 255/58

(54) **PRODUCTION PROCESS OF L-PHENYLALANINE DERIVATIVES BY MICROORGANISMS**
PROZESS FÜR DIE HERSTELLUNG VON L-PHENYLALANIN-DERIVATEN DURCH MIKROORGANISMEN
PROCEDE DE PRODUCTION DE DERIVES DE L-PHENYLALANINE AU MOYEN DE MICRO-ORGANISMES

(30) Priority: 21.08.2000 JP 2000249358; 21.12.2000 US 256908 P; 16.04.2001 JP 2001116892; 23.04.2001 US 285246 P
(43) Date of publication of application: 21.05.2003
(73) Proprietor: Showa Denko K.K., Tokyo 105-8518 (JP)
(72) Inventor: AOKI, Hirobumi, Central Research Laboratory, Chiba-shi, Chiba 267-0056 (JP)
(74) Representative: Strehl Schübel-Hopf & Partner
(86) International application number: PCT/JP2001/007156
(87) International publication number: WO 2002/016630

(56) References cited:
- EP-A- 0 152 235
- EP-A- 0 167 411
- GB-A- 1 265 773
- US-A- 3 767 528
- US-A- 4 562 151
- US-A- 5 981 239
- PATENT ABSTRACTS OF JAPAN vol. 012, no. 410 (C-540), 28 October 1988 (1988-10-28) -& JP 63 148992 A (ASAHI GLASS CO LTD), 21 June 1988 (1988-06-21) cited in the application

## Description

### Technical Field.

The present invention relates to a process for producing an optically active L-phenylalanine derivative, which is obtained by utilizing the activity of a microorganism . More specifically, the present invention relates to a production process of adding ammonia to a cinnamic acid derivative and thereby obtaining a corresponding optically active L-phenylalanine derivative. The optically active L-phenylalanine derivative obtained by the process of the present invention is useful as a starting material or intermediate in the synthesis of pharmaceuticals, agrochemicals and other fine chemicals.

### Background Art

With respect to the process for obtaining an optically active amino acid, many studies have been made on the organic synthetic reaction using an asymmetric catalyst or on the fermentative production process utilizing the specificity of microorganisms.

One of the production processes for obtaining an optically active phenylalanine is a process of allowing phenylalanine ammonia-lyase, which is an enzyme of microorganisms, to act. on a cinnamic acid and thereby optical-selectively obtaining an optically active phenylalanine (British Patent 1489468 and JP-A-53-96388). (The term "JP-A" as used herein means an "unexamined published Japanese patent application".) This process utilizes an enzymatic reaction of adding an amino group to -carbon of a cinnamic acid under the action of an enzyme in a system of high ammonia concentration, where L-phenylalanine can be produced with high optical purity.

For obtaining an optically active L-phenylalanine derivative having a substituent on the phenyl group, a process of introducing a substituent on the phenyl group of L-phenylalanine using various modification reactions may be thought out. This process, however, inevitably incurs decrease in the yield or purity due to a side reaction in the part other than the phenyl group, and decrease in the optical purity due to progress of racemization under severe reaction conditions. Furthermore, since positional specificity of the substituent introduced on the phenyl group cannot be easily obtained, this process suffers from low yield and difficulty in separation/purification and is not practical.

To overcome this, a process of utilizing the above-described phenylalanine ammonia-lyase and allowing an enzyme to act on a cinnamic acid derivative which has a substituted phenyl group having a desired substituent previously introduced, may be thought out. The enzymatic reaction proceeds in moderate conditions and therefore, the phenyl group and the substituent on the phenyl group can be prevented from harmful effect by a side reaction, as a result, it is duly expected that an optically active L-phenylalanine derivative can be obtained with high purity. However, the phenylalanine ammonia-lyase generally has a strict substrate specificity and in many cases, exhibits extremely low or almost no reactivity with a substrate having a substituted phenyl group as compared with that in the original reaction of obtaining L-phenylalanine from a cinnamic acid. Only a few cases have been disclosed, for example, a process of using a cinnamic acid derivative having a fluorinated phenyl group as a starting material and obtaining an optically active fluorinated phenylalanine under the action of an enzyme (see, JP-A-63-148992) and a process of obtaining a phenylalanine derivative using a specific microorganism of the genus Rhodotorula (see, U.S. Patent 5,981,239).

EP 0 152 235 A2 discloses a process for producing L-phenylalanine using for example a microorganism belonging to the genus *Eurotium, Pellicularia (Thanatephorus)* or *Gonatobotryum.*

US 4,562,151 discloses the production L-phenylalanine and derivatives thereof, wherein L-phenylalanine ammonia-lyases from *Rhodotorula* or *Sporobolomyces* are reacted on cinnamic acid derivatives.

EP 0 167 411 A2 discloses the production of phenylalanine by reacting cinnamic acid with ammonia in the presence of a phenylalanine ammonia-lyase obtained from a microorganism belonging e.g. to the species *Cladosporium.*

### Problems to be Solved by the Invention

One of the objects of the present invention is to provide a novel process for simply and easily obtaining highly optical-pure phenylalanine derivatives having a substituent starting from cinnamic acid derivatives having a substituent on the phenyl group using microorganisms.

### Disclosure of the Invention

The present inventors have made extensive investigations in the screening of novel soil microorganisms and in the reactivity of known microorganisms producing phenylalanine ammonia-lyase so as to find a microorganism having an ammonia-lyase activity with a high reactivity for a cinnamic acid derivative having a substituent on the phenyl group. As a result, microorganisms belonging to the genus Cladosporium, the genus Eurotium, the genus Thanatephorus or the genus Gonatobotryum have been found to have capability of converting cinnamic acid derivatives having various substituted phenyl groups into a corresponding L-phenylalanine derivative. The present invention has been accomplished based on this finding.

It has not been known that microorganisms belonging to the genus Cladosporium, the genus Eurotium, the genus Thanatephorus and the genus Gonatobotryum have capability of converting various cinnamic acid derivatives having various substituted phenyl groups into a corresponding L-phenylalanine derivative, and the present inventors have found the capability for the first time.

The present invention provides a process for producing L-phenylalanine derivatives below:
1. A process for producing an L-phenylalanine derivative represented by the following general formula (1) : 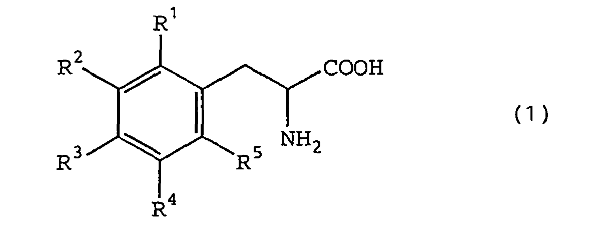 (wherein R¹, R², R³, R⁴ and R⁵ each independently represents hydrogen, a hydroxy group, an alkyl group (a linear or branched alkyl group having from 1 to 4 carbon atoms), an alkoxy group (the alkyl group constituting the alkoxy group is a linear or branched alkyl group having from 1 to 4 carbon atoms), a cyano group, a nitro group, a halogen, NR⁶R⁷ (wherein R⁶ and R⁷ each independently represents hydrogen or a linear or branched alkyl group having from 1 to 4 carbon atoms, or R⁶ and R⁷ may form a ring having from 3 to 5 carbon atoms and this ring may contain hetero atom), or a phenyl group which may have a substituent, provided that R¹, R², R³, R⁴ and R⁵ are not hydrogen at the same time), which comprises utilizing at least one microorganism belonging to any one of the genus Cladosporium, the genus Eurotium, the genus Thanatephorus and the genus Gonatobotryum , an ingredient of the microorganism or a material treated with the microorganism in the presence of ammonia and a cinnamic acid derivative represented by the following formula (2): 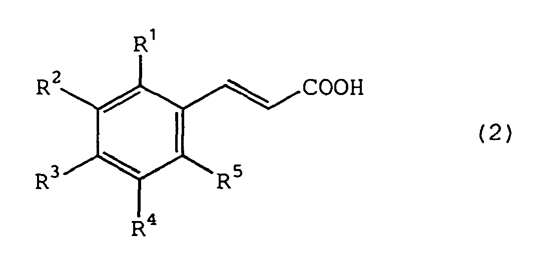 wherein the symbols have the same meanings as defined above.
2. The process for producing an L-phenylalanine derivative as described in 1, wherein R¹, R², R³, R⁴ and R⁵ in formula (1) are each independently hydrogen, a cyano group or a hydroxy group but are not hydrogen at the same time.
3. The process for producing an L-phenylalanine derivative as described in any one of above 1 or 2, using the microorganism belonging to any one of the genus Cladosporium, the genus Eurotium, the genus Thanatephorus and the genus Gonatobotryum , which is previously cultured in a culture medium containing a phenylalanine or phenylalanine derivative.
4. The process for producing an L-phenylalanine derivative as described in above 1, wherein at least a part of ammonia is supplied in the form of a carbonate.
5. The process for producing an L-phenylalanine derivative as described in 1 or 3, wherein the pH of the reacting solution is adjusted from 8.5 to 11 using carbon dioxide.
6. The process for producing an L-phenylalanine derivative as described in 1, wherein at least a part of the obtained L-phenylalanine derivative is recovered as an ammonium salt.
7. The process for producing an L-phenylalanine derivative as described in 4, 5 or 16, wherein at least a part of the obtained L-phenylalanine derivative is recovered as a carbonate.
8. The process for producing an L-phenylalanine derivative as described in 1, comprising reacting the microorganisms, ingredient of the microorganism or material treated with the microorganism in the solution of a cinnamic acid derivative having a content of 5% by mass or less.
9. The process for producing an L-phenylalanine derivative as described in any one of 1 or 2, wherein the L-phenylalanine derivative is 3-cyano-L-phenylalanine.
10. The process for producing an L-phenylalanine derivative as described in any one of 1 or 2, wherein the L-phenylalanine derivative is 4-cyano-L-phenylalanine.
11. The process for producing an L-phenylalanine derivative as described in any one of 1 or 2, wherein the L-phenylalanine derivative is 3-hydroxy-L-phenylalanine.
12. The process for producing an L-phenylalanine derivative as described in any one of 1 or 2, wherein the L-phenylalanine derivative is 4-hydroxy-L-phenylalanine.
13. The process for producing an L-phenylalanine derivative as described in any one of 1 or 2, wherein the L-phenylalanine derivative is 3,4-dihydroxy-L-phenylalanine.
14. The process for producing an L-phenylalanine derivative as described in 1 or 3, wherein the microorganism used is any one of Cladosporium colocasiae, Eurotium chevalieri, Thanatephorus cucumeris and Gonatobotryum apiculatum
15. The process for producing an L-phenylalanine derivative as described in 1 or 3, wherein the microorganism used is any one of Cladosporium colocasiae IFO 6698, Eurotium chevalieri IFO 4090, Thanatephorus cucumeris IFO 6254 and Gonatobotryum apiculatum IFO 9098
16. The process according to 5, wherein the pH adjustment using carbonic acid or an ammonium salt thereof is carried out by bubbling of carbon dioxide in a reaction solution.

### Mode for Carrying Out the Invention

The present invention is described in detail below.

The genus Cladosporium, the genus Eurotium, the genus Thanatephorus and the genus Gonatobotryum applied to the present invention are a kind of mold fungus widely discovered in the natural world. Examples of the microorganism strains showing the phenylalanine ammonia-lyase activity of catalyzing this reaction include the strains Cladosporium colocasiae IFO6698, Eurotium chevalieri IFO 4090, Thanatephorus cucumeris IFO 6254 and Gonatobotryum apiculatum IFO 9098, deposited with the Institute for Fermentation, Osaka. Among the microorganisms for use in the present invention, the genus Thanatephorus was formerly classified as the genus Pellicularia, and Thanatephorus cucumeris and Pellicularia filamentosa are the same microorganism.

To speak specifically, the reaction can be achieved as follows. For example, the strain Eurotium chevalieri IFO 4090 is cultured by a commonly known microorganism-culturing process, e.g., in a nutrient culture medium of 1% peptone at a temperature of approximately from 15 to 35°C, preferably approximately from 18 to 30°C, for approximately from 24 hours to 7 days. To the obtained culture solution, a cinnamic acid derivative having a substituent as a reaction starting material is added to a concentration of approximately from 1 ppm to 20%, preferably approximately from 10 ppm to 10%, ammonia is added to a final concentration of approximately from 0.5 to 11 M, preferably approximately from 1 to 9 M, and the pH is adjusted to approximately from 8.5 to 11, preferably approximately from 9 to 10.5. Subsequently, stirring is continued for approximately from 1 to 200 hours at the above described temperature. Examples of the acid which can be used to adjust the pH include inorganic acids such as sulfuric acid, hydrochloric acid, phosphoric acid, boric acid and carbonic acid, organic acids such as formic acid, acetic acid and propionic acid, and salts thereof. At this time, use of a volatile acid is advantageous in that the product can be simply and easily collected by subjecting the reaction solution to removal of cells and distillation, and the desalting step can be dispensed with. This acid is suitably carbonic acid. In this case, the carbonic acid includes carbonic acid produced when carbon dioxide is dissolved in an aqueous solution with bubbling. A salt of the above-described acid with ammonia may also be used as an ammonia source to the reaction solution and from the reasons described above, a part or the whole of ammonia source is suitably ammonium carbonate or ammonium hydrogencarbonate.

The added cinnamic acid derivative having a substituent may not be necessarily dissolved in the whole amount, however, a solvent, a surfactant may also be added so as to improve the solubility or dispersibility in the reaction solution. According to the consumption of cinnamic acid derivative with the progress of reaction, a cinnamic acid derivative may be added continuously or intermittently and in this case, the concentration of cinnamic acid derivative in the reaction solution is not limited to the above-described range. However, the cinnamic acid derivative added in excess is admitted to work as an inhibitor in the enzymatic reaction and therefore, in the major part of the reaction period, the concentration of the cinnamic acid derivative dissolved in the reaction solution preferably does not exceed the maximum 5%.

Examples of the carbon source which can be used in the culture medium for culturing the microorganism include saccharides such as glucose, sucrose, fructose and blackstrap molasses, organic substances such as ethanol, acetic acid, citric acid, succinic acid, lactic acid, benzoic acid and fatty acid, alkali metal salts of these organic substances, aliphatic hydrocarbons such as n-paraffin, and natural organic substances such as peptone, meat extract, fish extract, soy flour, bran, malt extract, and potato extract. These can be used individually or in combination, at a concentration of generally from 0.01 to 30%, preferably from 0.1 to 10%.

Examples of the nitrogen source which can be used in the culture medium for culturing the microorganism include inorganic nitrogen compounds such as ammonium sulfate, ammonium phosphate, sodium nitrate and potassium nitrate, nitrogen-containing organic substances such as urea and uric acid, and natural organic substances such as peptone, meat extract, fish extract, soy flour, malt extract and potato extract. These can be used individually or in combination, at a concentration of generally from 0.01 to 30%, preferably from 0.1 to 10%.

If desired, a phosphate such as potassium dihydrogenphosphate, or a metal salt such as magnesium sulfate, ferrous sulfate, calcium acetate, manganese chloride, copper sulfate, zinc sulfate, cobalt sulfate and nickel sulfate, may be added so as to improve the growth of the microorganism. The concentration added varies depending on the culture conditions but is generally from 0.01 to 5% for phosphate, from 10 ppm to 1% for magnesium salt and approximately from 0.1 to 1,000 ppm for other compounds. Furthermore, depending the culture medium selected, for example, a yeast extract, a casamino acid and a yeast nucleic acid may be added as the source for supplying vitamins, amino acid and nucleic acid, to a concentration of 1 to 100 ppm, whereby the growth of microorganism can be improved.

In order to improve the reactivity of the microorganism with the cinnamic acid derivative, it is useful to add approximately from 10 ppm to 1% of phenylalanine as a source for deriving phenylalanine ammonia-lyase during the culturing.

Whichever ingredient is used, the culture medium is preferably adjusted to a pH of 4.5 to 8, more preferably from 5 to 7.5. The microorganism cells previously cultured in the above-described culture medium are collected from the culture solution by a method such as centrifugal separation or membrane filtration and used for the reaction, and this is useful because impurities carried over from the culture solution can be reduced and the collection of product in the later stage can be facilitated.

The phenylalanine derivative generated in the reaction solution is collected by a method commonly used, which is selected depending on the state in the reaction solution, such as centrifugal separation, membrane filtration, drying under reduced pressure, distillation, solvent extraction, salting out, ion exchange and various chromatography analyses. The collection is simply achieved as follows. For example, the cell or a substance treated with the microorganism is removed from the reaction solution by filtration and centrifugal separation, the unreacted starting material cinnamic acid derivative is removed by solvent extraction or by adjusting the reaction solution to be acidic, and then the precipitate is removed. The pH of the obtained supernatant is again adjusted to the vicinity of the isoelectric point of the phenylalanine derivative and the precipitated derivative is recovered in the same manner. As such, the product can be recovered form the reaction solution in a high yield and a high purity. It is also useful to previously remove excess ammonia from the reaction solution by distillation or the like or to remove water by distillation to elevate the concentration and thereby improve the recovery of the product at the isoelectric point.

More simply, the pH of the reaction solution is adjusted using a volatile acid. In the case where the conversion percentage is sufficiently high, the product can be isolated as it is after the removal of cells and in the case where the starting material cinnamic acid derivative remains in a large amount, the reaction solution is rendered acidic and extracted by a solvent, or the precipitate resulting from the acidic condition is removed by filtration/centrifugal separation and thereafter, water, acid or base are removed by distillation, whereby the product can be isolated as a salt of phenylalanine derivative. The volatile acid or salt used in this method is suitably carbonic acid or an ammonium salt thereof .

Depending on the properties of the reaction product, the reaction rate may decrease due to accumulation of the product in the reaction solution. In such a case, a method of adding ammonia-containing water, physiological saline or reaction buffer solution according to the concentration of the product and thereby continuously diluting the reaction solution is suitably used. Also, the reaction rate may be recovered by a method of collecting the cells at the point when the reaction rate is decreased, recovering the supernatant as a product solution and returning again the collected cells to the solution containing the reaction starting material or to the suspension. These methods can be repeated many times insofar as the ammonia-lyase activity of the microorganism is maintained.

The present invention can also be similarly performed using a material treated with the microorganism which can be applied in the present invention, such as acellular extract or concentrated or extracted ingredient capable of catalyzing the above-described reaction from the acellular extract. Furthermore, the present invention can be achieved by immobilizing a microorganism which can be applied to the reaction, an extract solution or an extracted ingredient thereof, to a sparingly soluble support and contacting this immobilized matter with the starting material solution. Examples of the support for use in this immobilization include polyacrylamide, polyvinyl alcohol, poly-N-vinylformamide, polyallylamine, polyethyleneimine, methyl cellulose, glucomannan, alginate, carrageenan and copolymerized or crosslinked product thereof, which is a compound to form a sparingly water-soluble solid including the microorganism or an extracted ingredient thereof. These may be used individually or in combination. Furthermore, a material previously formed as a solid, such as activated carbon, porous ceramic, glass fiber, porous polymer molded article and nitrocellulose film, may be used after holding thereon the microorganism or an extract solution or extracted ingredient thereof. By continuously performing the reaction using such an immobilized matter, the inhibition of enzymatic reaction due to accumulation of the product can be effectively avoided.

The cinnamic acid derivative having a substituent, used as a starting material in the present invention is represented by the following formula (2): 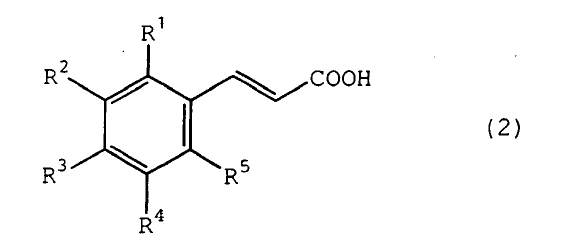 wherein R¹, R², R³, R⁴ and R⁵ each independently represents hydrogen, a hydroxyl group, an alkyl group (a linear or branched alkyl group having from 1 to 4 carbon atoms), an alkoxy group (the alkyl group constituting the alkoxy group is a linear or branched alkyl group having from 1 to 4 carbon atoms), a cyano group, a nitro group, a halogen such as chlorine or fluorine, NR⁶R⁷ (wherein R⁶ and R⁷ each independently represents hydrogen or a linear or branched alkyl group having from 1 to 4 carbon atoms or R⁶ and R⁷ may form a ring having from 3 to 5 carbon atoms and this ring may contain a hetero atom) or a phenyl group which may have a substituent, provided that R¹, R², R³, R⁴ and R⁵ are not necessarily hydrogen at the same time. Preferably, R¹, R², R³, R⁴ and R⁵ each independently is hydrogen, a cyano group, a hydroxyl group (provided that all are not hydrogen at the same time).

Preferred examples thereof include 3-cyanocinnamic acid, 4-cyanocinnamic acid, 3-hydroxycinnamic acid, 4-hydroxycinnamic acid and 3,4-dihydroxy cinnamic acid.

These cinnamic acid derivatives having a substituent, used as a starting material in the reaction, can be easily prepared by a method using a so-called Perkin reaction where acetic anhydride and the aldehyde group of a benzaldehyde derivative having introduced thereinto a corresponding substituent are allowed to act (see, Arch. Pharm. (Weinheim), 327(10), pp. 619-625 (1994)), a method of allowing a malonic acid to act in a pyridine solvent in the presence of piperidine (see, Journal of Chemical Society, pp. 357-360 (1939)), or various improved methods (see, Synth. Commun., 29(4), pp. 573-581 (1999)) or the like.

The L-phenylalanine derivative having a substituent, as the product of the present invention, is represented by the following formula (1): 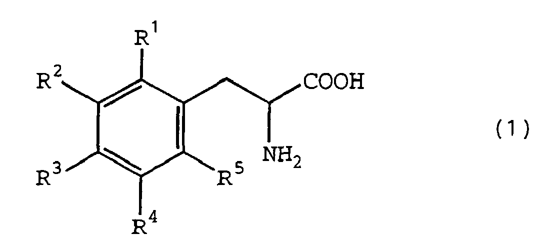 wherein R¹, R², R³, R⁴ and R⁵ each independently represents hydrogen, a hydroxy group, an alkyl group (a linear or branched alkyl group having from 1 to 4 carbon atoms), an alkoxy group (the alkyl group constituting the alkoxy group is a linear or branched alkyl group having from 1 to 4 carbon atoms), a cyano group, a nitro group, a halogen such as chlorine or fluorine, NR⁶R⁷ (wherein R⁶ and R⁷ each independently represents hydrogen or a linear or branched alkyl group having from 1 to 4 carbon atoms, or R⁶ and R⁷ may form a ring having from 3 to 5 carbon atoms and this ring may contain hetero atom), or a phenyl group which may have a substituent, provided that R¹, R², R³, R⁴ and R⁵ are not hydrogen at the same time. Preferably, R¹, R², R³, R⁴ and R⁵ each independently is hydrogen, a cyano group or a hydroxyl group (but all are not hydrogen at the same time).

Preferred examples thereof include 3-cyano-L-phenylalanine, 4-cyano-L-phenylalanine, 3-hydroxy-L-phenylalanine (metatyrosine), 4-hydroxy-L-phenylalanine (tyrosine) and 3,4-dihydroxy-L-phenylalanine (DOPA).

The L-phenylalanine derivative obtained in the present invention has a high optical purity and in the measuring method shown in Examples, exhibits an optical purity of 100% within the range of 0.1% as a detection limit.

In the present invention, the substituent on the phenyl group, such as cyano group or hydroxyl group, is not affected by the moderate conditions of this reaction and remains until the completion of reaction and accordingly, a desired corresponding L-phenylalanine derivative can be obtained in a high yield at a conversion percentage of nearly 100%.

Therefore, according to the process of the present invention, a high optical-purity L-phenylalanine derivative can be obtained by a one-step reaction using, as a substrate, a cinnamic acid derivative having a substituent, which can be simply and easily obtained by organic synthesis. The thus-obtained L-phenylalanine derivative having a substituent is useful as an intermediate for synthesizing an organic compound in the field of fine chemicals where high optical purity is required, such as pharmaceutical and agrochemical preparations.

### Best Mode for Carrying Out the Invention

The present invention is described below by referring to the Examples. The scope of the present invention is by no means limited to these Examples.

The obtained L-phenylalanine derivative was separated and determined by reverse phase HPLC under the analysis conditions shown below.

| | |
|---|---|
| Column: | Shodex^{®} RSpak NN-614 (manufactured by Showa Denko K.K.) |
| Column temperature: | 40°C |
| Eluent: | acetonitrile/water/aqueous solution of 50 mM H₃PO₄-KH₂PO₄ (pH 3) = 20/70/10 |
| Flow rate: | 1.0 ml/min |
| Detection: | by the absorption of UV at 210 nm |

The optical purity of the obtained L-phenylalanine derivative was analyzed by optical resolution HPLC under the conditions shown below.

| | |
|---|---|
| Column: | Shodex^{®} ORpak CRX-853 |
| Column temperature: | room temperature (22°C) |
| Eluent: | acetonitrile/water=15/85 CuSO₄ 2.5mM |
| Flow rate: | 1.0 ml/min |
| Detection: | by the absorption of UV at 256 nm |

### Example 1

Cladosporium colocasiae IFO6698 (divided from the Institute for Fermentation, Osaka) was inoculated into a potato dextrose agar medium (produced by Difco) and cultured in a constant temperature bath at 25°C for 72 hours. The formed microorganism cells were scraped and suspended in 100 mL of a liquid medium having the following composition in a 500 mL-volume baffled flask.

### <Culture Medium Composition>

| | |
|---|---|
| Glucose | 10 g |
| Peptone | 5 g |
| Yeast extract | 3 g |
| Malt extract | 3 g |
| L-Phenylalanine | 0.5 g |
| Distilled water | 1 L |
| pH=6.0 | |

The flask was placed in a constant temperature rotary shaking culture vessel at 25°C and the culture was performed at 150 rpm for 3 days. The obtained microorganism cells were recovered by centrifugation of 10,000 g and suspended in a 2M ammonium hydrogencarbonate/6M ammonia solution (prepared by dissolving ammonium hydrogencarbonate corresponding to the final concentration of 2M in a small amount of water, adding thereto concentrated aqueous ammonia corresponding to the final concentration of 6M, and filling it up with water, pH: 10.3) isovolumetric to the culture solution. To this cell suspension, 4-cyanocinnamic acid corresponding to 1% (weight/volume) was added, the flask was placed in a constant temperature rotary shaking culture vessel at 30°C, and the reaction was performed at 120 rpm for 96 hours.. Then, the reaction solution was subjected to reverse phase HPLC, as a result, accumulation of 4-cyano-L-phenylalanine of 0.58% was detected by the comparison with the sample. According to the gas chromatography analysis of the derivative, the optical purity thereof was calculated as 100% (detection limit: 0.1%).

### Example 2

Cladosporium colocasiae IFO 6698 was cultured in the same manner as in Example 1 and the obtained cells were suspended in a 2M ammonium hydrogencarbonate/6M ammonia solution (pH=10.3). To the cell suspension, 3-cyanocinnamic acid corresponding to 1% (w/v) was added, the flask was placed in a constant temperature rotary shaking culture vessel at 30°C, and the reaction was performed at 120 rpm for 96 hours. Then, the reaction solution was subjected to reverse phase HPLC, as a result, accumulation of 3-cyano-L-phenylalanine of 0.70% was detected by the comparison with the sample. According to the gas chromatography analysis of the derivative, the optical purity thereof was 100% (detection limit: 0.1%).

### Example 3

Cladosporium colocasiae IFO 6698 was cultured in the same manner as in Example 1 and the obtained cells were suspended in a 2M ammonium hydrogencarbonate/6M ammonia solution (pH=10.3). To the cell suspension, 4-hydroxy cinnamic acid corresponding to 1% (w/v) was added, the flask was placed in a constant temperature rotary shaking culture vessel at 30°C, and the reaction was performed at 120 rpm for 96 hours. Then, the reaction solution was subjected to reverse phase HPLC, as a result, accumulation of L-tyrosine of 0.33% was detected by the comparison with the sample. According to the gas chromatography analysis of the derivative, the optical purity thereof was 100% (detection limit: 0.1%).

### Example 4

Cladosporium colocasiae IFO 6698 was cultured in the same manner as in Example 1 and the obtained cells were suspended in a 2M ammonium hydrogencarbonate/6M ammonia solution (pH=10.3). To the cell suspension, 3-hydroxycinnamic acid corresponding to 1% (w/v) was added, the flask was placed in a constant temperature rotary shaking culture medium at 30°C, and the reaction was performed at 120 rpm for 96 hours. Then, the reaction solution was subjected to reverse phase HPLC, as a result, accumulation of metatyrosine of 0.80% was detected by the comparison with the sample. According to the gas chromatography analysis of the derivative, the optical purity thereof was 100% (detection limit: 0.1%).

### Example 5

Eurotium chevalieri IFO 4090 (divided from the Institute for Fermentation, Osaka) was inoculated in a potato dextrose agar medium (produced by Difco) and cultured in a constant temperature bath at 25°C for 72 hours. The formed microorganism cells were scraped and suspended in 100 mL of a liquid medium having the following composition in a 500 mL-volume baffled flask.

### <Culture Medium Composition>

| | |
|---|---|
| Glucose | 10 g |
| Peptone | 5 g |
| Yeast extract | 3 g |
| Malt extract | 3 g |
| L-Phenylalanine | 0.5 g |
| Distilled water | 1 L |
| pH=6.0 | |

The flask was placed in a constant temperature rotary shaking culture medium at 25°C and the culture were performed at 150 rpm for 5 days. From the obtained culture solution, the microorganism cells were recovered by centrifugation of 10,000 g and suspended in a 2M ammonium hydrogencarbonate/6M ammonia solution (prepared by dissolving ammonium hydrogencarbonate corresponding to the final concentration of 2M in a small amount of water, adding thereto a concentrated aqueous ammonia corresponding to the final concentration of 6M, and filling it up with water, pH: 10.3) isovolumetric to the culture solution. To this cell suspension, 4-cyanocinnamic acid corresponding to 1% (w/v) was added, the flask was placed in a constant temperature rotary shaking culture vessel at 30°C, and the reaction was performed at 120 rpm for 120 hours. Then, the reaction solution was subjected to reverse phase HPLC, as a result, accumulation of 4-cyano-L-phenylalanine of 0.22% was detected. According to the optical resolution HPLC analysis of the derivative, the optical purity thereof was calculated as 100% (detection limit: 0.1%).

### Example 6

Thanatephorus cucumeris IFO 6254 (divided from the Institute for Fermentation, Osaka) was inoculated in a potato dextrose agar culture medium (produced by Difco) and cultured in a constant temperature bath at 25°C for 72 hours. The formed microorganism cells were scraped and suspended in 100 mL of a commercially available potato dextrose broth (produced by Difco) prepared according to the manual of the manufacturer, and the suspension in a 500 mL-volume baffled flask was cultured in a constant temperature rotary shaking culture vessel at 25°C at 150 rpm for 5 days. From the obtained culture solution, the microorganism cells were recovered by centrifugation of 10,000 g and suspended in a 2M ammonium hydrogencarbonate/6M ammonia solution (pH: 10.3). To this cell suspension, 3-cyanocinnamic acid corresponding to 1% (w/v) was added, the flask was placed in a constant temperature rotary shaking culture medium at 30°C, and the reaction was performed at 120 rpm for 120 hours. Then, the reaction solution was subjected to reverse phase HPLC, as a result, accumulation of 3-cyano-L-phenylalanine of 0.39% was detected. According to the optical resolution HPLC analysis of the derivative, the optical purity thereof was 100% (detection limit: 0.1%).

### Example 7

Gonatobotryum apiculatum IFO 9098 (divided from the Institute for Fermentation, Osaka) was cultured in the same manner as in Example 5 and the obtained cells were suspended in a 2M ammonium hydrogencarbonate/6M ammonia solution (pH=10.3). To this cell suspension, 4-hydroxycinnamic acid corresponding to 1% (w/v) was added, the flask was placed in a constant temperature rotary shaking culture vessel at 30°C, and the reaction was performed at 120 rpm for 120 hours. Then, the reaction solution was subjected to reverse phase HPLC, as a result, accumulation of L-tyrosine of 0.32% was detected. According to the optical resolution HPLC analysis of the derivative, the optical purity thereof was 100% (detection limit: 0.1%).

### Example 8

Eurotium chevalieri IFO 4090 was cultured in the same manner as in Example 6 and then the obtained cells were suspended in a 2M ammonium hydrogencarbonate/6M ammonia solution (pH=10.3). To this cell suspension, 3-hydroxycinnamic acid corresponding to 1% (w/v) was added, the flask was placed in a constant temperature rotary shaking culture vessel at 30°C, and the reaction was performed at 120 rpm for 96 hours. Then, the reaction solution was subjected to reverse phase HPLC, as a result, accumulation of 3-hydroxy-L-phenylalanine (metatyrosine) of 0.64% was detected. According to the optical resolution HPLC analysis of the derivative, the optical purity thereof was 100% (detection limit: 0.1%).

### Example 9

50 mL of a reaction solution obtained in the same manner as in Example 1 was filtered by suction through filter paper to remove microorganism cells. The obtained aqueous solution was passed through carbon dioxide and thereby adjusted to a pH of 7, isovolumetric toluene was added thereto, and the resulting solution was stirred and extracted. The aqueous layer obtained by collection was removed by distillation under reduced pressure by means of an evaporator. The obtained solid product was analyzed by ¹H-NMR, element analysis and HPLC. As a result, the main ingredient of the solid was ammonium salt of 4-cyano-L-phenylalanine, the content thereof was 99.0% per dry weight, and the recovery of 4-cyano-L-phenylalanine from the reaction solution was 93%.

### Example 10

50 mL of a reaction solution obtained in the same manner as in Example 2 was filtered by suction through filter paper to remove microorganism cells. The obtained aqueous solution was passed through carbon dioxide and thereby adjusted to a pH of 7, isovolumetric toluene was added thereto, and the resulting solution was stirred and extracted. The aqueous layer obtained by collection was dried to solidification under reduced pressure by means of an evaporator. The obtained solid product was analyzed by ¹H-NMR, element analysis and HPLC. As a result, the main ingredient of the solid was 4-cyano-L-phenylalanine, the content thereof was 98.0% per dry weight, and the recovery of 4-cyano-L-phenylalanine from the reaction solution was 91.3%.

### Industrial Applicability

According to the present invention, L-phenylalanine derivatives having various substituents on the corresponding phenyl group can be simply and easily obtained with good efficiency, starting from cinnamic acid derivatives which can be easily synthesized.

## Claims

1. A process for producing an L-phenylalanine derivative represented by formula (1) 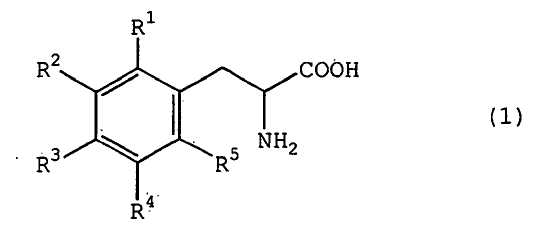 wherein R¹, R², R³, R⁴ and R⁵ each independently represents hydrogen, a hydroxy group, a linear or branched alkyl group having from 1 to 4 carbon atoms, an alkoxy group, whose alkyl group is a linear or branched alkyl group having from 1 to 4 carbon atoms, a cyano group, a nitro group, a halogen, NR⁶R⁷, wherein R⁶ and R⁷ each independently represents hydrogen or a linear or branched alkyl group having from 1 to 4 carbon atoms, or R⁶ and R⁷ may form a ring having from 3 to 5 carbon atoms and this ring may contain a hetero atom, or a phenyl group which may have a substituent, provided that R¹, R², R³, R⁴ and R⁵ are not hydrogen at the same time,
comprising reacting at least one microorganism belonging to any one of the genus Cladosporium, the genus Eurotium, the genus Thanatephorus and the genus Gonatobotryum , an ingredient of the microorganism or a material treated with the microorganism in the presence of ammonia and a cinnamic acid derivative represented by the following formula (2): 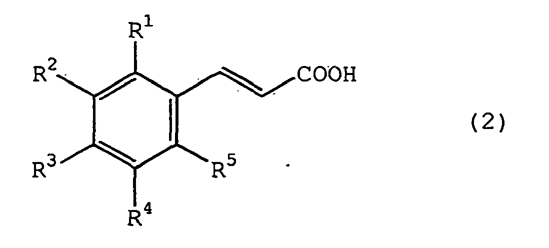 wherein the symbols have the same meanings as defined above.

2. The process for producing an L-phenylalanine derivative as claimed in claim 1, wherein R¹, R², R³, R⁴ and R⁵ in formula (1) are each independently hydrogen, a cyano group or a hydroxy group but are not hydrogen at the same time.

3. The process for producing an L-phenylalanine derivative as claimed in claim 1 or 2, using the microorganism belonging to any one of the genus Cladosporium, the genus Eurotium, the genus Thanatephorus and the genus Gonatobotryum , which is previously cultured in a culture medium containing a phenylalanine or phenylalanine derivative.

4. The process for producing an L-phenylalanine derivative as claimed in claim 1, wherein at least a part of ammonia is supplied in the form of a carbonate.

5. The process for producing an L-phenylalanine derivative as claimed in claim 1 or 3,
wherein the pH of the reaction solution is adjusted from 8.5 to 11 using carbonic acid or an ammonium salt thereof.

6. The process for producing an L-phenylalanine derivative as claimed in claim 1, wherein at least a part of the obtained L-phenylalanine derivative is recovered as an ammonium salt.

7. The process for producing an L-phenylalanine derivative as claimed in claim 4, 5 or 16,
wherein at least a part of the obtained L-phenylalanine derivative is recovered as a carbonate.

8. The process for producing an L-phenylalanine derivative as claimed in claim 1, comprising reacting the microorganisms, ingredient of the microorganism or material treated with the microorganism in the solution of a cinnamic acid derivative having a content of 5% by mass or less.

9. The process for producing an L-phenylalanine derivative as claimed in claim 1 or 2,
wherein the L-phenylalanine derivative is 3-cyano-L-phenylalanine.

10. The process for producing an L-phenylalanine derivative as claimed in claim 1 or 2,
wherein the L-phenylalanine derivative is 4-cyano-L-phenylalanine.

11. The process for producing an L-phenylalanine derivative as claimed in claim 1 or 2,
wherein the L-phenylalanine derivative is 3-hydroxy-L-phenylalanine.

12. The process for producing an L-phenylalanine derivative as claimed in claim 1 or 2,
wherein the L-phenylalanine derivative is 4-hydroxy-L-phenylalanine.

13. The process for producing an L-phenylalanine derivative as claimed in claim 1 or 2,
wherein the L-phenylalanine derivative is 3,4-dihydroxy-L-phenylalanine.

14. The process for producing an L-phenylalanine derivative as claimed in claim 1 or 3,
wherein the
microorganism used is any one of Cladosporium colocasiae, Eurotium chevalieri, Thanatephorus cucumeris and Gonatobotryum apiculatum.

15. The process for producing an L-phenylalanine derivative as claimed in claim 1 or 3,
wherein the microorganism used is any one of Cladosporium colocasiae IFO 6698, Eurotium chevalieri IFO 4090, Thanatephorus cucumeris IFO 6254 and Gonatobotryum apiculatum IFO 9098.

16. The process for producing an L-phenylalanine derivative as claimed in claim 5, wherein the pH. adjustment using carbonic acid or an ammonium salt thereof is carried out by bubbling of carbon dioxide in an reaction solution.

## Patentansprüche

1. Verfahren zur Herstellung eines L-Phenylalaninderivats der Formel (1) 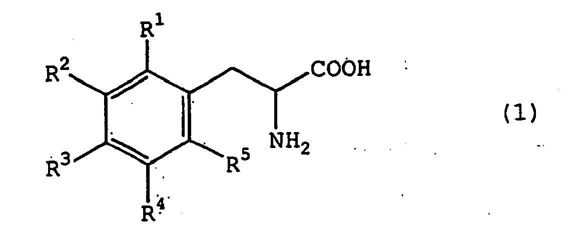 worin R¹, R², R³, R⁴ und R⁵ jeweils unabhängig Wasserstoff, eine Hydroxygruppe, eine lineare oder verzweigte Alkylgruppe mit 1 bis 4 Kohlenstoffatomen, eine Alkoxygruppe, deren Alkylgruppe eine lineare oder verzweigte Alkylgruppe mit 1 bis 4 Kohlenstoffatomen ist, eine Cyanogruppe, eine Nitrogruppe, ein Halogen, NR⁶R⁷, worin R⁶ und R⁷ jeweils unabhängig Wasserstoff oder eine lineare oder verzweigte Alkylgruppe mit 1 bis 4 Kohlenstoffatomen darstellen oder R⁶ und R⁷ einen Ring mit 3 bis 5 Kohlenstoffatomen bilden können und dieser Ring ein Heteroatom enthalten kann, oder eine Phenylgruppe, welche einen Substituenten aufweisen kann, darstellen, mit der Maßgabe, dass R¹, R², R³, R⁴ und R⁵ nicht gleichzeitig Wasserstoff sind,
umfassend das Reagierenlassen mindestens eines Mikroorganismus, der zu einer der Gattungen Cladosporium, Eurotium, Thanatephorus und Gonatobotryum gehört, eines Bestandteils des Mikroorganismus oder eines mit dem Mikroorganismus behandelten Materials in Gegenwart von Ammoniak und eines Zimtsäurederivats der folgenden Formel (2): 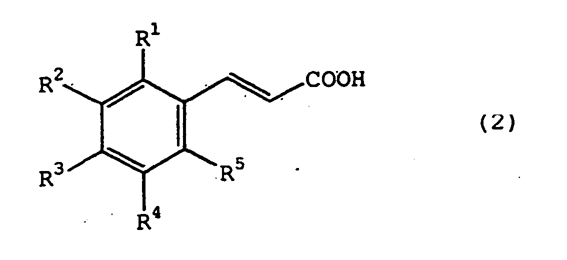 worin die Symbole die gleichen Bedeutungen wie oben definiert aufweisen.

2. Verfahren zur Herstellung eines L-Phenylalaninderivats nach Anspruch 1, worin R¹, R², R³, R⁴ und R⁵ in Formel (1) jeweils unabhängig Wasserstoff, eine Cyanogruppe oder eine Hydroxygruppe darstellen, jedoch nicht gleichzeitig Wasserstoff sind.

3. Verfahren zur Herstellung eines L-Phenylalaninderivats nach Anspruch 1 oder 2 unter Verwendung des Mikroorganismus, der zu einer der Gattungen Cladosporium, Eurotium, Thanatephorus und Gonatobotryum gehört, wobei dieser zuvor in einem Kulturmedium kultiviert wurde, welches ein Phenylalanin oder Phenylalaninderivat enthält.

4. Verfahren zur Herstellung eines L-Phenylalaninderivats nach Anspruch 1, worin zumindest ein Teil des Ammoniaks in Form eines Carbonats zugeführt wird.

5. Verfahren zur Herstellung eines L-Phenylalaninderivats nach Anspruch 1 oder 3, worin der pH der Reaktionslösung unter Verwendung von Kohlensäure oder eines Ammoniumsalzes davon von 8,5 auf 11 eingestellt wird.

6. Verfahren zur Herstellung eines L-Phenylalaninderivats nach Anspruch 1, worin mindestens ein Teil des erhaltenen L-Phenylalaninderivats als ein Ammoniumsalz gewonnen wird.

7. Verfahren zur Herstellung eines L-Phenylalaninderivats nach Anspruch 4, 5 oder 16, worin zumindest ein Teil des erhaltenen L-Phenylalaninderivats als ein Carbonat gewonnen wird.

8. Verfahren zur Herstellung eines L-Phenylalaninderivats nach Anspruch 1, umfassend das Reagierenlassen des Mikroorganismus, des Bestandteils des Mikroorganismus oder des mit dem Mikroorganismus behandelten Materials in der Lösung eines Zimtsäurederivats mit einem Gehalt von 5 Massen-% oder weniger.

9. Verfahren zur Herstellung eines L-Phenylalaninderivats nach Anspruch 1 oder 2, worin das L-Phenylalaninderivat 3-Cyano-L-phenylalanin ist.

10. Verfahren zur Herstellung eines L-Phenylalaninderivats nach Anspruch 1 oder 2, worin das L-Phenylalaninderivat 4-Cyano-L-phenylalanin ist.

11. Verfahren zur Herstellung eines L-Phenylalaninderivats nach Anspruch 1 oder 2, worin das L-Phenylalaninderivat 3-Hydroxy-L-phenylalanin ist.

12. Verfahren zur Herstellung eines L-Phenylalaninderivats nach Anspruch 1 oder 2, worin das L-Phenylalaninderivat 4-Hydroxy-L-phenylalanin ist.

13. Verfahren zur Herstellung eines L-Phenylalaninderivats nach Anspruch 1 oder 2, worin das L-Phenylalaninderivat 3,4-Dihydroxy-L-phenylalanin ist.

14. Verfahren zur Herstellung eines L-Phenylalaninderivats nach Anspruch 1 oder 3, worin der verwendete Mikroorganismus Cladosporium colocasiae, Eurotium chevalieri, Thanatephorus cucumeris oder Gonatobotryum apiculatum ist.

15. Verfahren zur Herstellung eines L-Phenylalaninderivats nach Anspruch 1 oder 3, worin der verwendete Mikroorganismus Cladosporium colocasiae IFO 6698, Eurotium chevalieri IFO 4090, Thanatephorus cucumeris IFO 6254 oder Gonatobotryum apiculatum IFO 9098 ist.

16. Verfahren zur Herstellung eines L-Phenylalaninderivats nach Anspruch 5, worin die pH-Einstellung unter Verwendung von Kohlensäure oder eines Ammoniumsalzes davon durch Einblasen von Kohlenstoffmonoxid in eine Reaktionslösung duchgeführt wird.

## Revendications

1. Procédé de production d'un dérivé de L-phénylalanine représenté par la formule (1) 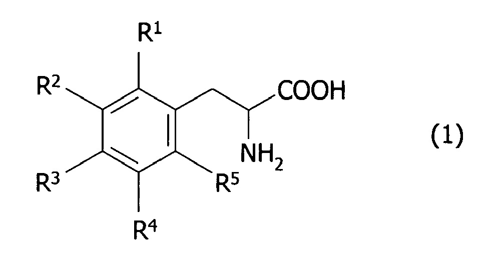 dans laquelle R¹, R², R³, R⁴ et R⁵ représentent chacun indépendamment un atome d'hydrogène, un groupe hydroxy, un groupe alkyle linéaire ou ramifié ayant de 1 à 4 atomes de carbone, un groupe alcoxy, dont le groupe alkyle est un groupe alkyle linéaire ou ramifié ayant de 1 à 4 atomes de carbone, un groupe cyano, un groupe nitro, un atome d'halogène, un groupe NR⁶R⁷, où R⁶ et R⁷ représentent chacun indépendamment un atome d'hydrogène ou un groupe alkyle linéaire ou ramifié ayant de 1 à 4 atomes de carbone, ou R⁶ et R⁷ peuvent former un cycle ayant de 3 à 5 atomes de carbone et ce cycle peut contenir un hétéroatome, ou un groupe phényle qui peut présenter un substituant à condition que R¹, R², R³, R⁴ et R⁵ ne sont pas un atome d'hydrogène simultanément,
comprenant la réaction d'au moins un micro-organisme appartenant à l'une quelconque de l'espèce Cladosporium, de l'espèce Eurotium, de l'espèce Thanatephorus et de l'espèce Gonatobotryum, d'un ingrédient du micro-organisme ou d'un matériau traité avec le micro-organisme en présence d'ammoniac et d'un dérivé d'acide cinnamique représenté par la formule (2) suivante : 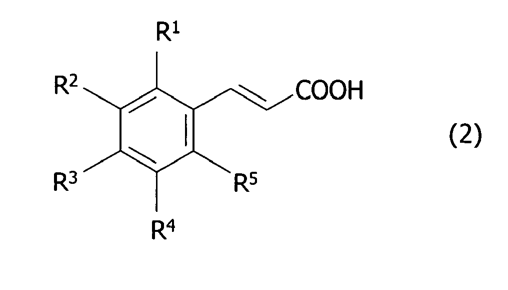 dans laquelle les symboles ont les mêmes significations que celles définies ci-dessus.

2. Procédé de production d'un dérivé de L-phénylalanine selon la revendication 1, dans lequel R¹, R², R³, R⁴ et R⁵ dans la formule (1) sont chacun indépendamment un atome d'hydrogène, un groupe cyano ou un groupe hydroxy mais ne sont pas un atome d'hydrogène simultanément.

3. Procédé de production d'un dérivé de L-phénylalanine selon la revendication 1 ou 2 utilisant le micro-organisme appartenant à l'une quelconque de l'espèce Cladosporium, de l'espèce Eurotium, de l'espèce Thanatephorus et de l'espèce Gonatobotryum, lequel est préalablement mis en culture dans un milieu de culture contenant une phénylalanine ou un dérivé de phénylalanine.

4. Procédé de production d'un dérivé de L-phénylalanine selon la revendication 1, dans lequel au moins une partie de l'ammoniac est fournie dans la forme d'un carbonate.

5. Procédé de production d'un dérivé de L-phénylalanine selon la revendication 1 ou 3, dans lequel le pH de la solution de réaction est ajusté de 0,5 à 11 en utilisant de l'acide carbonique ou un sel d'ammonium de celui-ci.

6. Procédé de production d'un dérivé de L-phénylalanine selon la revendication 1, dans lequel au moins une partie du dérivé de L-phénylalanine obtenu est récupérée dans la forme d'un sel d'ammonium.

7. Procédé de production d'un dérivé de L-phénylalanine selon la revendication 4, 5 ou 16, dans lequel au moins une partie du dérivé de L-phénylalanine obtenu est récupérée dans la forme d'un carbonate.

8. Procédé de production d'un dérivé de L-phénylalanine selon la revendication 1 comprenant la réaction du micro-organisme, de l'ingrédient du micro-organisme ou du matériau traité avec le micro-organisme dans la solution d'un dérivé d'acide cinnamique ayant une teneur de 5 % en masse ou inférieure.

9. Procédé de production d'un dérivé de L-phénylalanine selon la revendication 1 ou 2, dans lequel le dérivé de L-phénylalanine est la 3-cyano-L-phénylalanine.

10. Procédé de production d'un dérivé de L-phénylalanine selon la revendication 1 ou 2, dans lequel le dérivé de L-phénylalanine est la 4-cyano-L-phénylalanine.

11. Procédé de production d'un dérivé de L-phénylalanine selon la revendication 1 ou 2, dans lequel le dérivé de L-phénylalanine est la 3-hydroxy-L-phénylalanine.

12. Procédé de production d'un dérivé de L-phénylalanine selon la revendication 1 ou 2, dans lequel le dérivé de L-phénylalanine est la 4-hydroxy-L-phénylalanine.

13. Procédé de production d'un dérivé de L-phénylalanine selon la revendication 1 ou 2, dans lequel le dérivé de L-phénylalanine est la 3,4-dihydroxy-L-phénylalanine.

14. Procédé de production d'un dérivé de L-phénylalanine selon la revendication 1 ou 3, dans lequel le micro-organisme utilisé est l'un quelconque parmi Cladosporium colocasiae, Eurothim chevalieri, Thanatephorus cucumeris et Gonatobotryum apiculatum.

15. Procédé de production d'un dérivé de L-phénylalanine selon la revendication 1 ou 3, dans lequel le micro-organisme utilisé est l'un quelconque parmi Cladosporium colocasiae IFO 6698, Eurothim chevalieri IFO 4090, Thanatephorus cucumeris IFO 6254 et Gonatobotyum apiculatum IFO 9098.

16. Procédé de production d'un dérivé de L-phénylalanine selon la revendication 5, dans lequel on réalise l'ajustement du pH en utilisant de l'acide carbonique ou un sel d'ammonium de celui-ci par barbotage de dioxyde de carbone dans une solution de réaction.
